Europäisches Patentamt
European Patent Office
Office européen des brevets

(19)

(11) Veröffentlichungsnummer: **0 332 024 A1**

(12)

# EUROPÄISCHE PATENTANMELDUNG

(21) Anmeldenummer: **89103496.9**

(22) Anmeldetag: **28.02.89**

(51) Int. Cl.4: **C07D 285/12 , C07D 239/26 , C07D 213/30 , C07D 213/65 , C09K 19/34**

(30) Priorität: **10.03.88 DE 3807871**

(43) Veröffentlichungstag der Anmeldung: **13.09.89 Patentblatt 89/37**

(84) Benannte Vertragsstaaten: **ES GR**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Reiffenrath, Volker**
**Jahnstrasse 18**
**D-6101 Rossdorf(DE)**
Erfinder: **Krause, Joachim, Dr.**
**Samuel-Morse-Strasse 14**
**D-6110 Dieburg(DE)**
Erfinder: **Wächtler, Andreas, Dr.**
**Goethestrasse 34**
**D-6103 Griesheim(DE)**
Erfinder: **Geelhaar, Thomas, Dr.**
**Trajanstrasse 12**
**D-6500 Mainz(DE)**

(54) **Heterocyclische Derivate des 1,2-Difluorbenzols.**

(57) Heterocyclische Derivate des 1,2-Difluorbenzols der Formel I

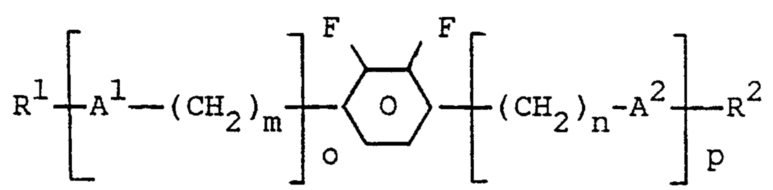

I

worin

$R^1$ und $R^2$ jeweils unabhängig voneinander eine Alkylgruppen mit 1-15 C-Atomen, worin auch eine oder mehrere $CH_2$-Gruppen durch einen bivalenten Rest ausgewählt aus der Gruppe bestehend aus -O-, -S-, -CO-, -O-CO-, -CO-O-, -CH=CH- und -C≡C-ersetzt sein können, wobei zwei Heteroatome nicht direkt miteinander verknüpft sind,

$A^1$ und $A^2$ jeweils unabhängig voneinander eine unsubstituierte oder durch Fluor ein- oder mehrfach substituierte 1,4-Phenylengruppe, worin auch eine oder zwei CH-Gruppen durch N ersetzt sein können, eine 1,3,4-Thiadiazol-2,5-diyl-Gruppe oder eine trans-1,4-Cyclohexylen-Gruppe,

m und n jeweils 0 oder 2,

o 0 oder 1, und

p 0, 1 oder 2 bedeutet,

wobei (o + p) 1, 2 oder 3 ist und im Falle p = 2 die Gruppen $A^2$ und n gleich oder verschieden sein können, mit der Maßgabe, daß mindestens eine der Gruppen $A^1$ und $A^2$ eine 1,3,4-Thiadiazol-2,5-diyl-Gruppe oder eine unsubstituierte oder substituierte 1,4-Phenylengruppe ist, worin eine oder zwei CH-Gruppen durch N ersetzt sind, eignen sich als Komponenten flüssigkristalliner Phasen.

EP 0 332 024 A1

## Heterocyclische Derivate des 1,2-Difluorbenzols

Die Erfindung betrifft Heterocyclische Derivate des 1,2-Difluorbenzols der Formel I

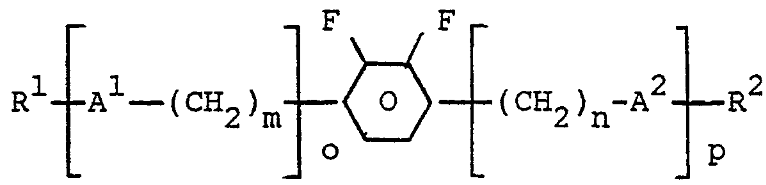

I

worin

$R^1$ und $R^2$ jeweils unabhängig voneinander eine Alkylgruppen mit 1-15 C-Atomen, worin auch eine oder mehrere $CH_2$-Gruppen durch einen bivalenten Rest ausgewählt aus der Gruppe bestehend aus -O-, -S-, -CO-, -O-CO-, -CO-O-, -CH=CH- und -C≡C- ersetzt sein können, wobei zwei Heteroatome nicht direkt miteinander verknüpft sind,

$A^1$ und $A^2$ jeweils unabhängig voneinander eine unsubstituierte oder durch Fluor ein- oder mehrfach substituierte 1,4-Phenylengruppe, worin auch eine oder zwei CH-Gruppen durch N ersetzt sein können, eine 1,3,4-Thiadiazol-2,5-diyl-Gruppe oder eine trans-1,4-Cyclohexylen-Gruppe,

m und n jeweils 0 oder 2,

o 0 oder 1, und

p 0, 1 oder 2 bedeutet,

wobei (o + p) 1, 2 oder 3 ist und im Falle p = 2 die Gruppen $A^2$ und n gleich oder verschieden sein können, mit der Maßgabe, daß mindestens eine der Gruppen $A^1$ und $A^2$ eine 1,3,4-Thiadiazol-2,5-diyl-Gruppe oder eine unsubstituierte oder substituierte 1,4-Phenylengruppe ist, worin eine oder zwei CH-Gruppen durch N ersetzt sind. Die Erfindung betrifft ferner flüssigkristalline Phasen enthaltend Verbindungen der Formel I, deren Verwendung als Komponenten flüssigkristalliner Phasen, sowie elektrooptische Anzeigeelemente enthalten derartige Phasen.

Der Einfachheit halber bedeuten im folgenden Cyc eine 1,4-Cyclohexylengruppe, Pyd eine Pyridin-2,5-diylgruppe, Pyr eine Pyrimidin-2,5-diylgruppe, Pyn eine Pyrazin-2,5-diylgruppe, Thi eine 1,3,4-Thiadiazol-2,5-diylgruppe und Phe eine 1,4-Phenylengruppe, wobei diese Gruppen unsubstituiert oder durch ein oder zwei Fluor substituiert sein kann. Vorzugsweise sind diese Gruppen unsubstituiert. $PheF_2$ bedeutet eine Gruppe der Formel

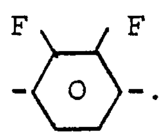

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Die Verbindungen der Formel I zeichnen sich durch eine deutlich negative Anisotropie der Dielektrizitätskonstante aus und werden in einem elektrischen Feld mit ihren Molekülländsachsen senkrecht zur Feldrichtung ausgerichtet.

Dieser Effekt ist bekannt und wird zur Steuerung der optischen Transparenz in verschiedenen Flüssigkristallanzeigen ausgenützt, so z.B. in Flüssigkristallzellen vom Lichtstreuungstyp (dynamische Streuung), vom sogenannten DAP-Typ (Deformation aufgerichteter Phasen) oder ECB-Typ (electrically controlled birefringence) oder vom Gast/Wirt-Typ (guest host interaction).

Weiterhin eignen sich Verbindungen der Formel I als Komponenten chiral getilteter smektischer Phasen. Chiral getiltete smektische flüssigkristalline Phasen mit ferroelektrischen Eigenschaften können hergestellt werden, in dem man Basis-Mischungen mit einer oder mehreren getilteten smektischen Phasen mit einem geeigneten chiralen Dotierstoff versetzt (L.A. Veresnev et al., Mol. Cryst. Liq. Cryst. 89, 327 (1982); H.R. Brand et al., J. Physique 44 (lett.), L-771 (1983). Solche Phasen können als Dielektrika für schnell schaltende Displays verwendet werden, die auf dem von Clark und Lagerwall beschriebenen Prinzip

der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen.

Es sind bisher bereits eine Reihe von flüssigkristallinen Verbindungen mit schwach negativer dielektrischer Anisotropie synthetisiert worden. Hingegen sind noch relativ wenig Flüssigkristallkomponenten mit großer negativer Anisotropie der Dielektrizitätskonstante bekannt. Zudem weisen letztere im allgemeinen Nachteile auf, wie z.B. schlechte Löslichkeit in Mischungen, hohe Viskosität, hohe Schmelzpunkte und chemische Instabilität. Es besteht daher ein Bedarf an weiteren Verbindungen mit negativer dielektrischer Anisotropie, die es erlauben, die Eigenschaften von Mischungen für verschiedenste elektro-optische Anwendungen weiter zu verbessern.

Flüssigkristallverbindungen mit negativer dielektrischer Anisotropie, welche zwei oder drei über Carboxylgruppen oder Kovalenzbindung verknüpfte Ringe und eine oder mehrere seitliche Gruppen, wie Halogen, Cyano oder Nitrogruppen aufweisen, sind bekannt aus DE 22 40 864, DE 26 13 293, DE 28 35 662, DE 28 36 086 und EP 023 728.

In der EP 084 194 werden in einer breiten Formel die hier beanspruchten Verbindungen umfaßt. Jedoch sind dort keine Einzelverbindungen der erfindungsgemäßen Formel genannt. Der Fachmann konnte somit aus dem Stand der Technik weder in einfacher Art und Weise Synthesemöglichkeiten für die beanspruchten Verbindungen entnehmen noch erkennen, daß die erfindungsgemäßen Verbindungen überwiegend günstig gelegene Mesophasenbereiche aufweisen und sich durch eine große negative Anisotropie der Dielektrizität bei gleichzeitiger niedriger Viskosität auszeichnen.

Auch fehlt dort jeglicher Hinweis auf die Möglichkeit des Einsatzes der erfindungsgemäßen Verbindungen in Displays, die auf der SSFLC-Technologie beruhen, da die dort beanspruchten Verbindungen geringe smektische Tendenzen aufweisen.

Weiterhin sind Dibenzoesäureester des 2,3-Dichlorhydrochinons bekannt (z.B. Bristol et al., J. Org. Chem. 39, 3138 (1974) oder Clanderman et al., J. Am. Chem. Soc. 97, 1585 (1975)), die allerdings monotrop sind bzw. sehr kleine Mesophasenbereiche aufweisen. Die von Eidenschink et al. beschriebenen Ester der 4-Hydroxy-2,3-dichlorbenzoesäure (Angew. Chem. 89, 103 (1977)) besitzen ebenfalls nur schmale Mesophasenbereiche.

Die aus der DE OS 29 33 563 bekannten 4-Alkyl-2,3-dichlorphenyl-4′-alkylbicyclohexyl-4-carbonsäureester erlauben wegen ihrer hohen Viskosität keine technische Anwendung.

Der Erfindung lag die Aufgabe zugrunde stabile, flüssigkristalline oder mesogene Verbindungen mit einer großen negativen Anisotropie der Dielektrizität und gleichzeitig geringer Viskosität aufzuzeigen.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit breitem Mesophasenbereich und vergleichsweise niedriger Viskosität herstellbar.

Weiterhin eignen sich die Verbindungen der Formel I als Komponenten chiral getilteter smektischer flüssigkristalliner Phasen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder die Phasenbereiche und/oder den Tiltwinkel und/oder den Pitch eines solchen Dielektrikums zu varriieren.

Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I, sowie Flüssigkristall-Anzeigeelemente, die derartige Phasen enthalten. Derartige Phasen weisen besonders vorteilhafte elastische Konstanten auf, und eignen sich wegen ihrer niedrigen $\Delta\epsilon/\epsilon_{\perp}$-Werte besonders für TFT-Mischungen.

Vor- und nachstehend haben $R^1$, $R^2$, $A^1$, $A^2$, m und p die angegebene Bedeutung, sofern nicht

ausdrücklich etwas anderes vermerkt ist.

Die Verbindungen der Formel I umfassen dementsprechend Verbindungen mit 2 Ringen der Teilformel Ia und Ib, Verbindung mit 3 Ringen der Teilformel Ic bis Ih und Verbindungen mit 4 Ringen der Teilformel Ij bis Im:

| | |
|---|---|
| $R^1$-PheF$_2$-$A^2$-$R^2$ | Ia |
| $R^1$-PheF$_2$-$CH_2CH_2$-$A^2$-$R^2$ | Ib |
| $R^1$-PheF$_2$-$A^2$-$A^2$-$R^2$ | Ic |
| $R^1$-PheF$_2$-$A^2$-$CH_2CH_2$-$A^2$-$R^2$ | Id |
| $R^1$-PheF$_2$-$CH_2CH_2$-$A^2$-$A^2$-$R^2$ | Ie |
| $R^1$-$A^1$-PheF$_2$-$A^2$-$R^2$ | If |
| $R^1$-$A^1$-$CH_2CH_2$-PheF$_2$-$A^2$-$R^2$ | Ig |
| $R^1$-$A^1$-$CH_2CH_2$-PheF$_2$-$CH_2CH_2$-$A^2$-$R^2$ | Ih |
| $R^1$-$A^1$-PheF$_2$-$A^2$-$A^2$-$R^2$ | Ij |
| $R^1$-$A^1$-PheF$_2$-$CH_2CH_2$-$A^2$-$A^2$-$R^2$ | Ik |
| $R^1$-$A^1$-$CH_2CH_2$-PheF$_2$-$A^2$-$A^2$-$R^2$ | Il |
| $R^1$-$A^1$-PheF$_2$-$A^2$-$CH_2CH_2$-$A^2$-$R^2$ | Im |

Darunter sind diejenigen der Teilformeln Ia, Ic, Id, If und Ig besonders bevorzugt.

In den Verbindungen der vor- und nachstehenden Formeln bedeuten $R^1$ und $R^2$ vorzugsweise Alkyl oder Alkoxy.

Weiterhin bevorzugt sind Verbindungen der vor- und nachstehenden Formeln, in denen einer der Reste $R^1$ und $R^2$, Alkenyl oder Oxaalkyl (z.B. Alkoxymethyl) bedeutet.

$R^1$ und $R^2$ weisen in den vor- und nachstehenden Formeln vorzugsweise 2-12 C-Atome, insbesondere 3-10 C-Atome auf. In $R^1$ und $R^2$ können auch eine oder zwei $CH_2$-Gruppen ersetzt sein. Vorzugsweise ist nur eine $CH_2$-Gruppe ersetzt durch -O-, oder -CH=CH-.

In den vor- und nachstehenden Formeln bedeuten $R^1$ und $R^2$ vorzugsweise Alkyl, Alkoxy oder eine andere Oxaalkylgruppe, ferner auch Alkylgruppen, in denen eine oder zwei $CH_2$-Gruppen durch -CH=CH- ersetzt sein können.

Falls $R^1$ und $R^2$ Alkylreste, in denen auch eine ("Alkoxy" bzw. "Oxaalkyl") oder zwei ("Alkoxylakoxy" bzw. "Dioxaalkyl") nicht benachbarte $CH_2$-Gruppen durch O-Atome ersetzt sein können, bedeuten, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxypropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4-, 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Falls $R^1$ und $R^2$ einen Alkylrest bedeuten, in dem eine $CH_2$-Gruppe durch -CH = CH- ersetzt ist, so ist die transForm bevorzugt. Dieser Alkenylrest kann geradkettig oder verzweigt sein. Vorzugsweise ist er geradkettig und hat 2-10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formel I mit verzweigten Flügelgruppen $R^1$ und/oder $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste $R^1$ und/oder $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy.

Formel I umfaßt sowohl die Racemate dieser Verbindungen als auch die optischen Antipoden sowie deren Gemische.

m und n sind vorzugsweise 0. Ferner bevorzugt sind Verbindungen der Formel I, die eine $-CH_2CH_2-$ Gruppe enthalten, d.h. m oder n ist einmal 1.

(o + p) ist vorzugsweise 1 oder 2. Falls o = 0 und $R^1 \neq$ n-Alkoxy, ist (o + p) vorzugsweise 2 oder 3.

Die Gruppen $A^1$ und $A^2$ bedeuten vorzugsweise Cyc, Phe, Pyd, Pyr, Pyn oder Thi. Vorzugsweise ist nur eine der Gruppen $A^1$ und $A^2$ Cyc, Pyd, Pyr, Pyn oder Thi.

Besonders bevorzugte Bedeutungen von $\left[-A^1-(CH_2)_m\right]_o-PheF_2\left[(CH_2)_n-A^2\right]_p$ sind die folgenden Gruppen 1 bis 28:

| | |
|---|---|
| Pyd-PheF$_2$ | 1 |
| Pyr-PheF$_2$ | 2 |
| Pyn-PheF$_2$ | 3 |
| Thi-PheF$_2$ | 4 |
| Pyd-PheF$_2$-Phe | 5 |
| Pyr-PheF$_2$-Phe | 6 |
| Pyn-PheF$_2$-Phe | 7 |
| Thi-PheF$_2$-Phe | 8 |
| Pyd-Phe-PheF$_2$ | 9 |
| Pyr-Phe-PheF$_2$ | 10 |
| Pyn-Phe-PheF$_2$ | 11 |
| Thi-Phe-PheF$_2$ | 12 |
| Phe-Pyd-PheF$_2$ | 13 |
| Phe-Pyr-PheF$_2$ | 14 |
| Phe-Pyn-PheF$_2$ | 15 |
| Phe-Thi-PheF$_2$ | 16 |
| PheF$_2$-Pyd-Cyc | 17 |
| PheF$_2$-Pyr-Cyc | 18 |
| PheF$_2$-Pyn-Cyc | 19 |
| PheF$_2$-Thi-Cyc | 20 |
| Pyd-PheF$_2$-Cyc | 21 |
| Pyr-PheF$_2$-Cyc | 22 |
| Pyn-PheF$_2$-Cyc | 23 |
| Thi-PheF$_2$-Cyc | 24 |
| Pyd-PheF$_2$-CH$_2$CH$_2$-Cyc | 25 |
| Pyr-PheF$_2$-CH$_2$CH$_2$-Cyc | 26 |
| Pyn-PheF$_2$-CH$_2$CH$_2$-Cyc | 27 |
| Thi-PheF$_2$-CH$_2$CH$_2$-Cyc | 28 |

Unter den Verbindungen der Formeln I sowie Ia bis Im sind diejenigen bevorzugt, in denen mindestens einer der darin enthaltenen Reste eine der angegebenen bevorzugten Bedeutungen hat.

Eine kleinere Gruppe von besonders bevorzugten stoffen ist diejenige der Formeln:

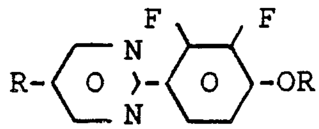

A

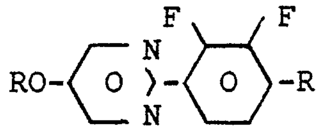

B

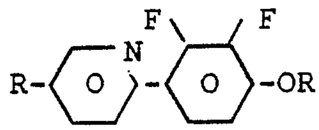

C

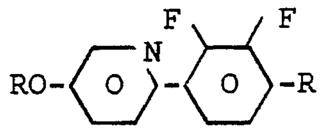

D

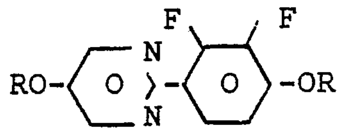

E

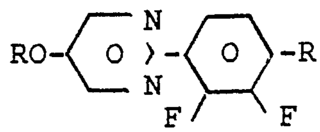

F

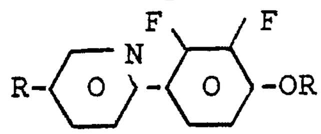

G

R bedeutet in diesen Formeln jeweils unabhängig voneinander geradkettiges oder einfach verzweigtes (vorzugsweise Methylverzweigung) Alkyl mit 3 bis 12 C-Atomen, worin eine nicht mit O verknüpfte $CH_2$-Gruppe auch durch -O- oder -CH=CH- ersetzt sein kann.

R ist vorzugsweise Alkyl, Oxaalkyl oder Alkenyl, Oxaalkyl oder Alkenyl mit vorzugsweise 3 bis 12, insbesondere mit 5 bis 12 C-Atomen. Die Gruppen R sind vorzugsweise geradkettig.

Besonders bevorzugt sind erfindungsgemäße Verbindungen der Teilformel I2

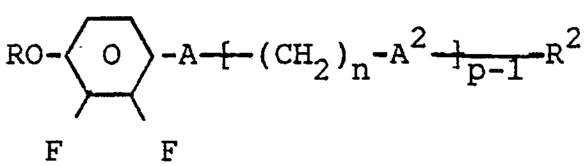

I2

worin R eine Alkylgruppe mit 1 bis 15 C-Atomen ist, worin auch eine oder mehrere $CH_2$-Gruppen durch einen bivalenten Rest ausgewählt aus der Gruppe bestehend aus -O-, -S-, -CO-, -O-CO-, -CO-O-, -CH=CH- und -C≡C- ersetzt sein können, wobei zwei Heteroatome nicht direkt miteinander verknüpft sind. p ist 1 oder 2. n, $A^2$ und $R^2$ haben die oben angegebene Bedeutung. A ist eine 1,3,4-Thiadiazol-2,5-diyl-, Pyrimidin-2,5-diyl-, Pyridin-2,5-diyl oder Pyrazin-2,6-diyl-Gruppe. Vorzugsweise ist A

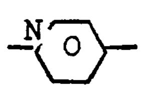 oder 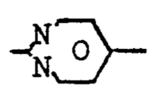.

Eine besonders bevorzugte kleinere Gruppe von Verbindungen ist diejenige der Formel I1

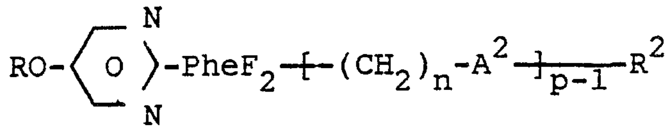

I1

worin R eine Alkylgruppe mit 1 bis 15 C-Atomen ist, worin auch eine oder mehrere $CH_2$-Gruppen durch einen bivalenten Rest ausgewählt aus der Gruppe bestehend aus -O-, -S-, -CO-, -O-CO-, -CO-O-, -CH=CH- und -C≡C- ersetzt sein können, wobei zwei Heteroatome nicht direkt miteinander verknüpft sind. p ist 1 oder 2. $PheF_2$ n, $A^2$ und $R^2$ haben die oben angegebene Bedeutung. Die Verbindungen der Formel I1 können wie im folgenden Schema 1 angegeben hergestellt werden:

Schema 1

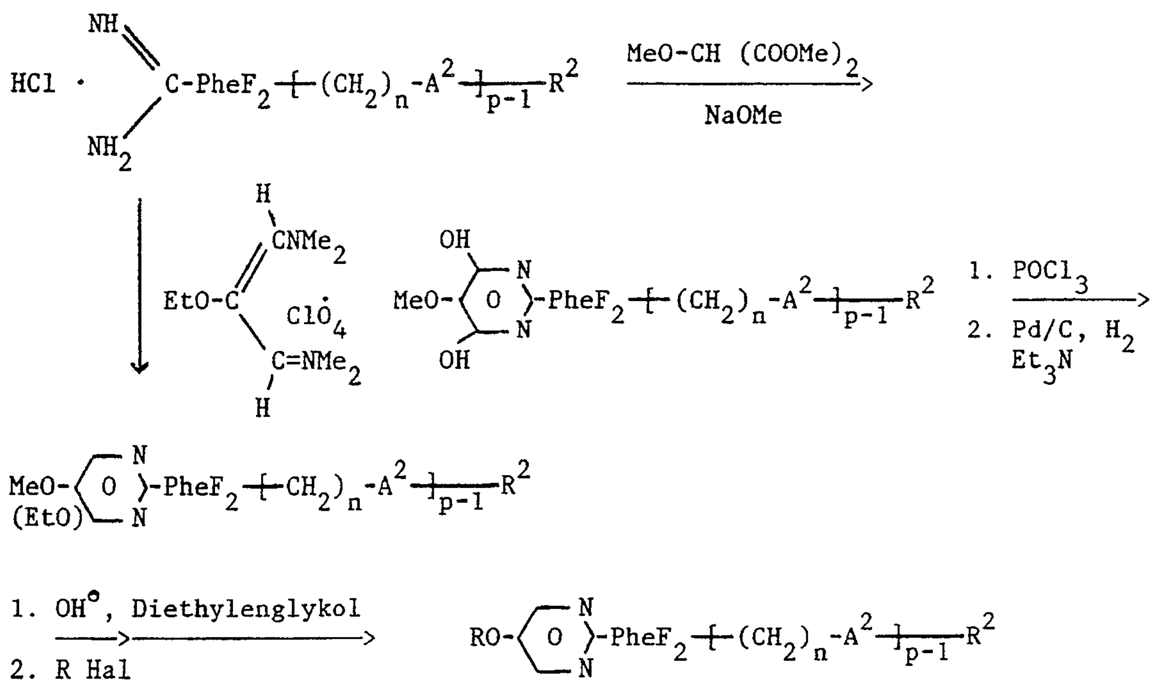


Synthesemöglichkeiten für weitere bevorzugte Verbindungen sind in den folgenden Schemata angegeben:

Schema 2

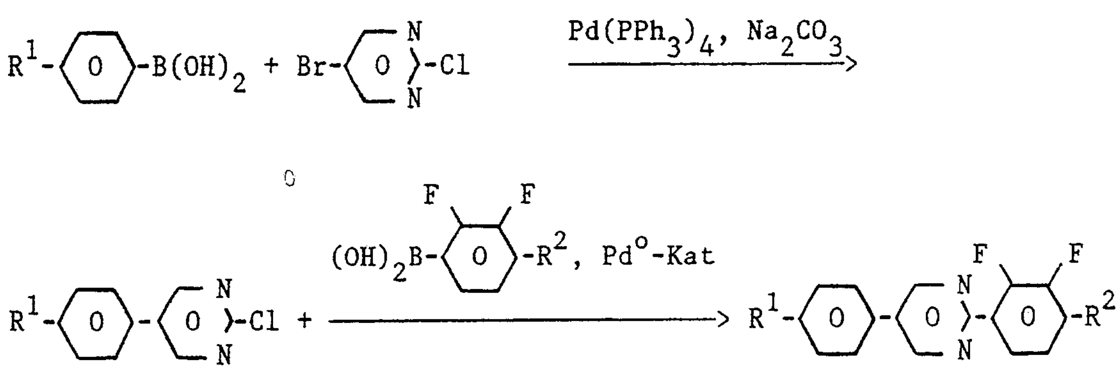

Schema 3

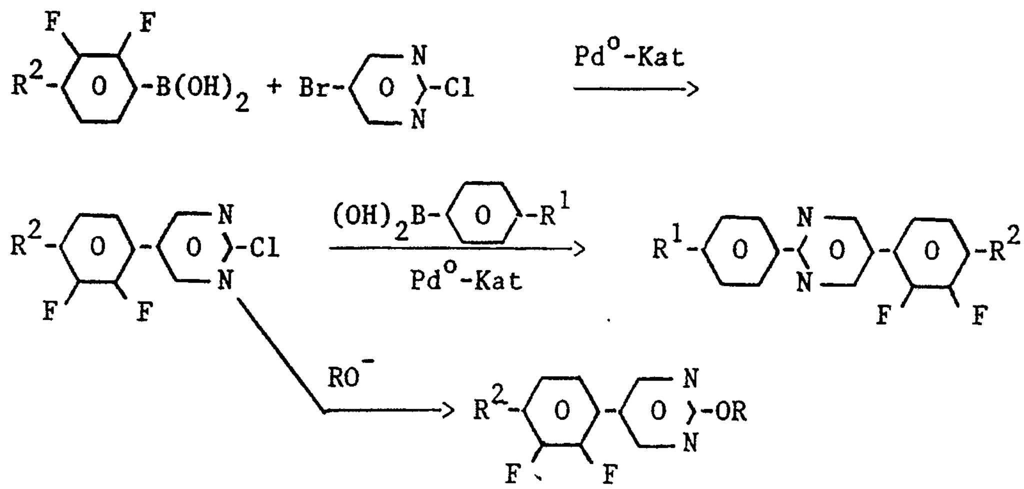


Schema 4

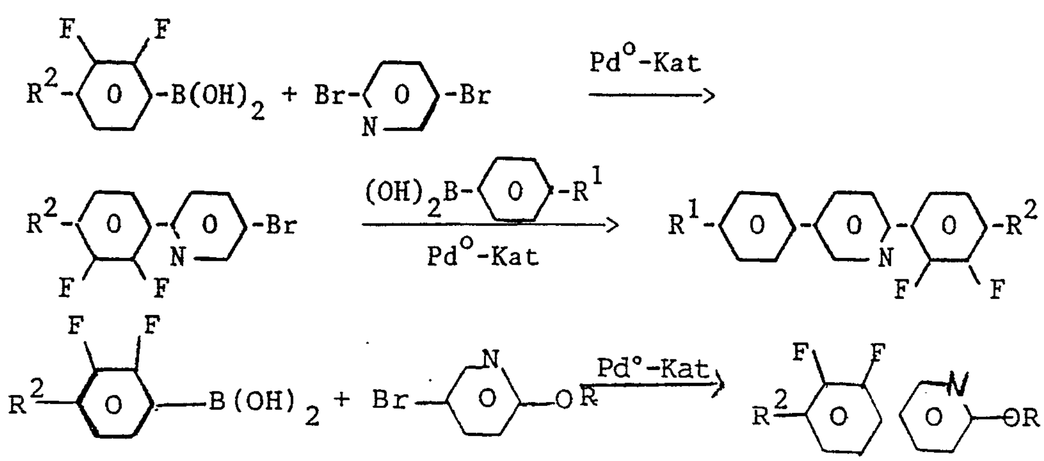


Schema 5

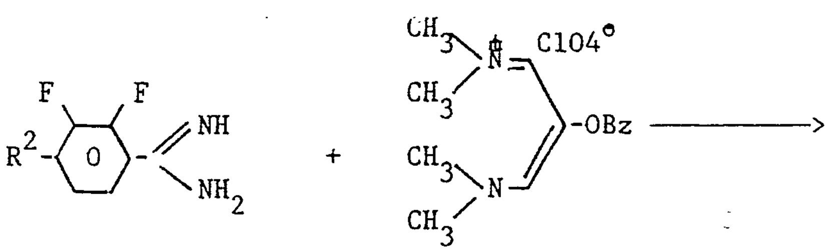

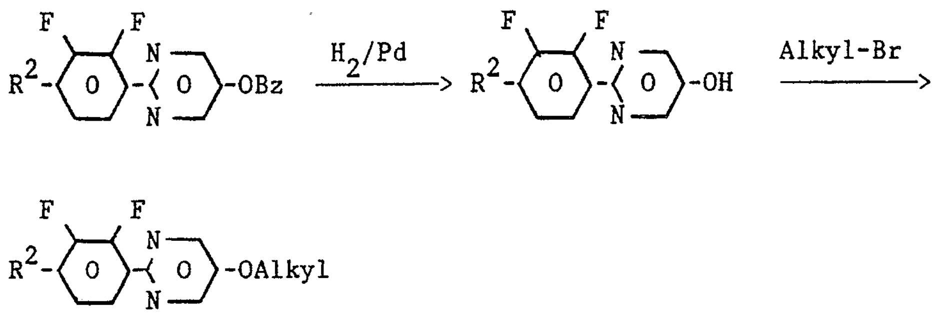


Schema 6

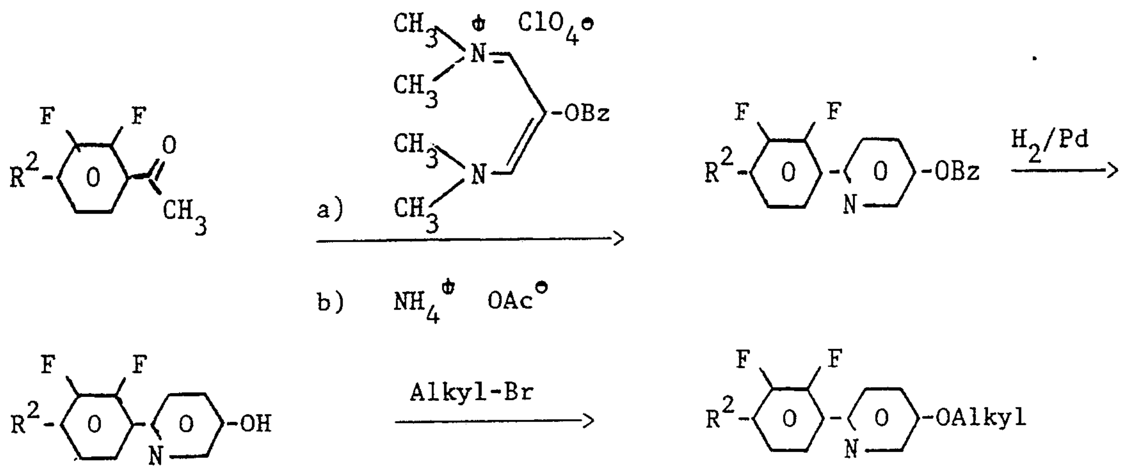


Schema 7

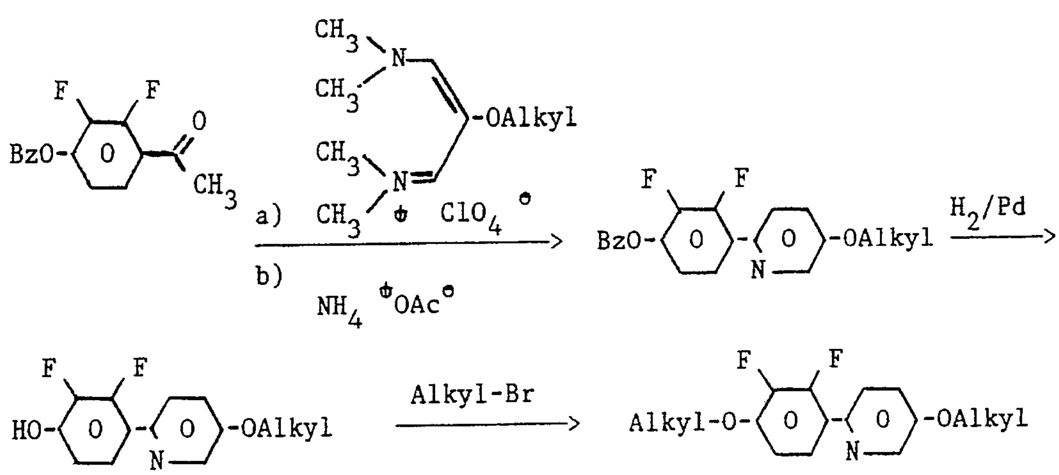


Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I bzw. deren Vorstufen sind ausgehend von 1,2-Difluorbenzol zugänglich. Dieses wird nach bekanntem Verfahren (z.B. A.M. Roe et al., J. Chem. Soc. Chem. Comm., 22, 582 (1965)) metalliert und mit dem entsprechenden Elektrophil umgesetzt. Mit dem so erhaltenen 1-substituierten 2,3-Difluorbenzol läßt sich diese Reaktionssequenz ein zweites Mal mit einem geeigneten Elektrophil durchführen und man gelangt so zu den für die Synthesen der Heterocyclen geeigneten 1,4-disubstituierten 2,3-Difluorbenzolen. 1,2-Difluorbenzol bzw. 1-substituiertes 2,3-Difluorbenzol wird in einem inerten Lösungsmittel wie Diethylether, Tetrahydrofuran, Dimethoxyethan, tert-Butylmethylether oder Dioxan, Kohlenwasserstoffen wie Hexan, Heptan, Cyclohexan, Benzol oder Toluol oder Gemischen dieser Lösungsmittel gegebenenfalls unter Zusatz eine Komplexierungsmittels wie Tetramethylethylendiamin (TMEDA) oder Hexamethylphosphorsäuretriamid mit Phenyllithium, Lithiumtetramethylpiperidin, n-, sek- oder tert-Butyllithium bei Temperaturen von -100 °C bis +50 °C vorzugsweise -78 °C bis 0 °C umgesetzt.

Die Lithium-2,3-difluorphenyl-Verbindungen werden bei -100 °C bis 0 °C vorzugsweise bei -50 °C mit den entsprechenden Elektrophilen umgesetzt. Geeignete Elektrophile sind Aldehyde, Ketone, Nitrile, Epoxide, Carbonsäure-Derivate wie Ester, Anhydride oder Halogenide, Halogenameisensäureester oder Kohlendioxid. Weitere Einzelheiten können der DOS 38 07 910 entnommen werden.

Zur Umsetzung mit aliphatischen oder aromatischen Halogen-Verbindungen werden die Lithium-2,3-difluorphenyl-Verbindungen transmetalliert und unter Übergangsmetallkatalyse gekoppelt. Besonders geeignet sind hierfür die Zink- (vgl. DE OS 36 32 410) oder die Titan-2,3-difluorphenyl-Verbindungen (vgl. DE OS 37 36 489).

Die Einführung der heterocyclischen Strukturelemente kann einerseits dadurch erfolgen, daß man Vorstufen, die diese Strukturelemente bereits enthalten nach den bekannten Methoden zu den Verbindungen der Formel I umsetzt. Andererseits können aber auch in entsprechend strukturierten Vorstufen oder Unterstruktureinheiten der Verbindungen der Formel I nach an sich bekannten Methoden Heterocyclenreste erzeugt werden.

So können beispielsweise 2,5-disubstituierte 1,3,4-Thiadiazole durch Umsetzung von N,N′-Diacylhydrazinen mit üblichen Thiierungsreagenzien wie $P_4S_{10}$ oder Lawesson's Reagenz hergestellt werden. Die N,N′-Diacylhydrazine ihrerseits sind nach bekannten Methoden aus der entsprechenden Carbonsäuren zugänglich, wobei die Carbonsäuren mit einem 2,3-Difluor-1,4-phenylen-Strukturelement wie vorstehend beschrieben durch Umsetzung entsprechender metallisierter Vorstufen mit Kohlendioxid erhalten werden können.

Die 2,5-disubstituierten Pyrimidine können beispielsweise durch Umsetzung entsprechender Amidinhydrochloride (herstellbar aus den entsprechenden Carbonsäuren) mit Malondialdehydtetramethylacetalen nach an sich bekannten Methoden hergestellt werden. Die 2,5-disubstituierten Pyridine sind durch Kopplung von metallorganischen Zinkverbindungen mit entsprechenden Brompyridinderivaten entsprechend DE-OS 36 32 410 erhältich. Die 2,5-disubstituierten Pyrazine sind erhältlich durch Kondensation von geeignet substituierten Ethylendiaminen mit Glyoxalderivaten, Oxidation der Dihydroverbindungen mit Luftsauerstoff oder anderen Oxidationsmitteln und Isolierung der gewünschten 2,5-disubstituierten Pyrazine aus dem entstandenen Gemisch der 2,5- und 2,6-Disubstitutionsprodukte. Die 3,6-disubstituierten Pyridazine sind zugänglich durch Umsetzung von 1,4-Diketonen (hergestellt z.B. nach Stetter durch thiazoliumsalzkatalysierte Addition eines Aldehyds an ein $\alpha,\beta$-ungesättigtes Keton) und anschliessende Oxidation des Dihydropyridazins mit Luftsauerstoff oder anderen Oxidationsmitteln wie Kaliumnitrit oder Chromsäure in Eisessig.

Im folgenden wird die Synthese einiger besonders interessanter Hydroxy-Zwischenstufen beschrieben:

a) 5-Alkyl-2-(2,3-difluor-4-hydroxyphenyl)-pyridine sind erhältlich durch Umsetzung von 2,3-Difluor-4-benzyloxy-benzamidinhydrochlorid mit 2-Alkyl-3-ethoxy-acroleinen bzw. mit in 2-Stellung alkylierten Malonaldehydtetraacetalen oder entsprechend substituierten vinylogen Formamidiniumsalzen (R.M. Wagner und CH. Jutz, Chem. Ber. 104 2975 (1971), indem man vorzugsweise die Komponenten in DMF (Dimethylformamid) erhitzt und anschließend die Schutzgruppe abspaltet.

b) 5-Hydroxy-2-(2,3-difluor-4-alkylphenyl)-pyrimidine bzw. 5-Hydroxy-2(2,3-difluor-4-alkoxyphenyl)-pyrimidine sind erhältlich durch Kondensation von 4-Alkyl- bzw. 4-Alkoxy-2,3-difluorbenzamidinhydrochlorid mit 2-Benzyloxytrimethiniumperchlorat (A. Holy, Z. Arnold; Collection Czechoslov. Chem. Comm. 38 1371-1380 (1973), oder 2-Benzyloxy-3-dimethylaminoacrolein (H. Horstmann et al., Arzneimittelforsch. 11 682 (1961) und anschließender Hydrogenolyse der Benzylgruppe.

c) 5-Hydroxy-2(2,3-difluor-4-alkylphenyl)pyridine bzw. 5-Hydroxy-2(2,3-difluor-4-alkoxyphenyl)pyridine sind erhältlich aus 2-Benzyloxytrimethiniumsalz durch Kondensation mit 4-Alkyl- oder 4-Alkoxy-2,3-difluoracetophenonen, Umsetzung mit $NH_3/NH_4Cl$ oder Ammoniumacetat.

Analog den Vorschriften von Ch. Jutz et al. (Liebigs Ann. Chem. 1975 874-900) und anschließende Hydrogenolyse oder aus 4-Alkyl- bzw. 4-Alkoxy-2,3-difluorphenylboronsäure durch Kopplung mit 5-Acetoxy-2-brompyridin (erhältlich aus 5-Hydroxy-2-brompyridin durch Veresterung) in Gegenwart eines Pd-Katalysators entsprechend den Arbeiten von Suzuki et al. (Synth. Commun. 11 513-19 (1981)).

d) 5-Alkoxy-2(2,3-difluor-4-hydroxyphenyl)pyridine sind erhältlich durch Kopplung von 2,3-Difluor-4-benzyloxyphenylboronsäure mit 5-Alkoxy-2-brompyridin entsprechend oben genannter Literatur und anschließende Hydrogenolyse.

e) 5-Alkyl-2(2,3-difluor-4-hydroxyphenyl)pyridine sind erhältlich durch Kopplung von 2-Brom-5-methylpyridin mit 2,3-Difluor-4-benzyloxyphenylboronsäure und einen Pd-Katalysator unter den bereits genannten Bedingungen, Kettenverlängerung der Methylgruppe durch Deprotonierung mit LDA als Base (-65 °C) und Alkylierung mit einem Alkylbromid und Hydrogenolyse.

f) 5-Alkyl-2(2,3-difluor-4-hydroxyphenyl)pyrimidine bzw. 5-Alkoxy-2(2,3-difluor-4-hydroxyphenyl)-pyrimidine sind herstellbar durch die übliche Kondensation von 2,3-Difluor-4-benzyloxybenzamidin mit 2-Alkylmalonaldehydtetraacetalen oder 2-Alkyl-3-ethoxyacroleinen bzw. 2,3-Dialkoxyacroleinen oder der entsprechenden Immoniumsalze oder Alkoxytrimethiniumsalzen und anschließender Hydrogenolyse.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4′-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclophexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1,2, 3, 4 und 5 charakterisieren:

$R'$-L-E-$R''$ 1

$R'$-L-COO-E-$R''$ 2

$R'$-L-OOC-E-$R''$ 3

$R'$-L-$CH_2CH_2$-E-$R''$

$R'$-L-C≡C-E-$R''$ 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe-, und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

$R'$ und $R''$ bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind $R'$ und $R''$ voneinander verschieden, wobei einer dieser Reste meist Alkyl oder Alkenyl ist. In den Verbindungen der Teilformeln 1b, 2b, 3b, 4b und 5b bedeutet $R''$ -CN, $-CF_3$, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden

oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:
K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1:

1,0 mol 2,3-Difluor-4-octyloxvbenzoylchlorid (herstellbar durch Alkylierung von 2,3-Difluorphenol mit Octylbromid/Kaliumcarbonat in Dimethylformamid (DMF), Metallierung des 2,3-Difluoroctyloxybenzols in 4-Stellung mit Butyllithium/Tetramethylethylendiamin in Tetrahydrofuran (THF) bei -70 bis -80°, Umsetzung mit Trockeneis, und Umsetzung der Säure mit Thionylchlorid) in 150 ml Pyridin werden unter Rühren bei 20-25° mit 0,1 mol Decansäurehydrazid versetzt und 2 Stunden nachgerührt. Man gießt in 750 ml Wasser, saugt ab, wäscht mit Wasser nach und trocknet.

Die Bisacylverbindung wird unter Erwärmen in der erforderlichen Menge THF gelöst und mit der 1,1-fachen molaren Menge Lawesson-Reagenz 3 Stunden unter Rückfluß erhitzt. Man destilliert den größten Teil des Lösungsmittels ab, versetzt mit Wasser und stellt das Gemisch mit Natronlauge alkalisch. Das ausgefallene 2-(2,3-Difluor-4-octyloxyphenyl)-5-nonyl-1,3,4-thiadiazol wird abgesaugt, mit Wasser gewaschen, getrocknet und aus Ethanol umkristallisiert.

Beispiel 2:

Analog vorhergehendem Beispiel wird durch Umsetzung von 4-Hexyloxybenzhydrazid mit 2,3-Difluor-4-heptylbenzoylchlorid (hergestellt durch Umsetzung von o-Difluorbenzol mit Butyllithium in Gegenwart von Kaliumtertiärbutylat bis -90 bis -100° in Tetrahydrofuran, Alkylierung der gebildeten Kaliumverbindung mit Heptylbromid/1,3-Dimethyltetrahydro-2-(1H)-pyrimidinon (DMPU), Isolierung des 2,3-Difluorheptylbenzols, erneute Metallierung mittels Butyllithium, anschließende Reaktion mit festem Kohlendioxid und Erhitzen der Säure mit Thionylchlorid) und anschließendem Ringschluß mit Lawesson-Reagenz 2-(2,3-Difluor-4-heptylbiphenyl)-5-(4-hexyloxyphenyl)-1,3,4-thiadiazol erhalten.

Analog werden hergestellt:
2-(2,3-Difluor-4-octyloxyphenyl)-5-nonyl-1,3,4-thiadiazol
2-(2,3-Difluor-4-nonyloxyphenyl)-5-nonyl-1,3,4-thiadiazol

2-(2,3-Difluor-4-decyloxyphenyl)-5-nonyl-1,3,4-thiadiazol
2-(2,3-Difluor-4-heptyloxyphenyl)-5-nonyl-1,3,4-thiadiazol
2-(2,3-Difluor-4-hexyloxyphenyl)-5-nonyl-1,3,4-thiadiazol
2-(2,3-Difluor-4-octyloxyphenyl)-5-octyl-1,3,4-thiadiazol
2-(2,3-Difluor-4-nonyloxyphenyl)-5-octyl-1,3,4-thiadiazol
2-(2,3-Difluor-4-decyloxyphenyl)-5-octyl-1,3,4-thiadiazol
2-(2,3-Difluor-4-heptyloxyphenyl)-5-octyl-1,3,4-thiadiazol
2-(2,3-Difluor-4-hexyloxyphenyl)-5-octyl-1,3,4-thiadiazol
2-(2,3-Difluor-4-octyloxyphenyl)-5-heptyl-1,3,4-thiadiazol
2-(2,3-Difluor-4-nonyloxyphenyl)-5-heptyl-1,3,4-thiadiazol
2-(2,3-Difluor-4-decyloxyphenyl)-5-heptyl-1,3,4-thiadiazol
2-(2,3-Difluor-4-heptyloxyphenyl)-5-heptyl-1,3,4-thiadiazol
2-(2,3-Difluor-4-hexyloxyphenyl)-5-heptyl-1,3,4-thiadiazol

Beispiel 3:

Durch Umsetzung von 2,3-Difluor-4′-pentylbiphenyl-4-carbonsäurechlorid und Valeriansäurehydrazid und anschließendem Ringschluß mit Lawesson-Reagenz erhält man 2-[2,3-Difluor-4-(4-pentylphenyl)-phenyl]-5-butyl-1,3,4-thiadiazol.

Der benötigte Biphenylester wird wie folgt erhalten:

Man lithiert o-Difluorbenzol bei -70 bis -80°, setzt bei dieser Temperatur mit 4-Pentylcyclohexanon um, dehydratisiert den entstandenen Alkohol und aromatisiert das Cyclohexenderivat durch Erhitzen mit 2,3-Dichlor-5,6-dicyan-p-benzochinon (DDQ) in Toluol unter Rückfluß. Das 2,3-Difluor-4′-pentylbiphenyl wird erneut metalliert und mit festem Kohlendioxid versetzt. Anschließend wird die Säure mit Methanol und Schwefelsäure verestert.

Beispiel 4:

2,3-Difluor-4-ethoxybenzoylchlorid(hergestellt wie in Beispiel 1 für die Octyloxyverbindung beschrieben) wird in Pyridin mit trans-4-Propylcyclohexancarbonsäurehydrazid und die Bisacylverbindung mit Lawesson-Reagenz in das 2-(2,3-Difluor-4-ethoxyphenyl)-5-(trans-4-propylcyclohexyl)-1,3,4-thiadiazol überführt.

Beispiel 5:

0,1 mol 2,3-Difluor-4-nonyloxybenzamidin-hydrochlorid (hergestellt aus 2,3-Difluor-4-nonyloxybenzoylchlorid durch Überführung in das Amid, dessen Dehydratisierung zum Nitril, Umsetzung des Nitrils mit Ethanol und Chlorwasserstoffgas und nachfolgende Reaktion des Imidoesters mit Ammoniak), 0,1 mol Nonylmalondialdehydtetramethylacetal und 50 ml DMF werden 12 Stunden auf 150° erhitzt. Anschließend wird das Reaktionsgemisch in Dichlormethan aufgenommen, mit Natriumhydrogencarbonatlösung und Wasser neutral gewaschen, getrocknet und das Lösungsmittel abdestilliert. Als Rückstand erhält man 2-(2,3-Difluor-4-nonyloxyphenyl)-5-nonylpyrimidin, das aus Ethanol umkristallisiert wird: C 41 $S_c$ 54 I.

Analog werden hergestellt:

2-(2,3-Difluor-4-decyloxyphenyl)-5-nonylpyrimidin
2-(2,3-Difluor-4-octyloxyphenyl)-5-nonylpyrimidin
2-(2,3-Difluor-4-heptyloxyphenyl)-5-nonylpyrimidin, C 47 $S_c$ 52 I
2-(2,3-Difluor-4-hexyloxyphenyl)-5-nonylpyrimidin
2-(2,3-Difluor-4-decyloxyphenyl)-5-decylpyrimidin
2-(2,3-Difluor-4-nonyloxyphenyl)-5-decylpyrimidin
2-(2,3-Difluor-4-octyloxyphenyl)-5-decylpyrimidin
2-(2,3-Difluor-4-heptyloxyphenyl)-5-decylpyrimidin
2-(2,3-Difluor-4-hexyloxyphenyl)-5-decylpyrimidin
2-(2,3-Difluor-4-decyloxyphenyl)-5-octylpyrimidin
2-(2,3-Difluor-4-nonyloxyphenyl)-5-octylpyrimidin
2-(2,3-Difluor-4-octyloxyphenyl)-5-octylpyrimidin
2-(2,3-Difluor-4-heptyloxyphenyl)-5-octylpyrimidin

2-(2,3-Difluor-4-hexyloxyphenyl)-5-octylpyrimidin
2-(2,3-Difluor-4-decyloxyphenyl)-5-heptylpyrimidin
2-(2,3-Difluor-4-nonyloxyphenyl)-5-heptylpyrimidin, K 46 $S_c$ (34) N (45) I
2-(2,3-Difluor-4-octyloxyphenyl)-5-heptylpyrimidin
2-(2,3-Difluor-4-heptyloxyphenyl)-5-heptylpyrimidin, K 36 $S_c$ (33) N 42 I
2-(2,3-Difluor-4-hexyloxyphenyl)-5-heptylpyrimidin
2-(2,3-Difluor-4-decyloxyphenyl)-5-hexylpyrimidin
2-(2,3-Difluor-4-nonyloxyphenyl)-5-hexylpyrimidin
2-(2,3-Difluor-4-octyloxyphenyl)-5-hexylpyrimidin
2-(2,3-Difluor-4-heptyloxyphenyl)-5-hexylpyrimidin
2-(2,3-Difluor-4-hexyloxyphenyl)-5-hexylpyrimidin

Beispiel 6:

Durch Umsetzung von 2,3-Difluor-4′-propyl-biphenyl-4-carbamidin-hydrochlorid (die dafür erforderliche Biphenylcarbonsäure wird analog Beispiel 3 erhalten) mit Butylmalondialdehyd-tetramethylacetal in DMF erhält man nach üblicher Aufarbeitung 2,3-Difluor-4-(5-butylpyrimidin-2-yl)-4′-propylbiphenyl.

Beispiel 7:

Durch Umsetzung von O-Difluorbenzol mit Butyllithium in Gegenwart von Kaliumtertiärbutylat bei -90 bis -100° in Tetrahydrofuran, Alkylierung der gebildeten Kaliumverbidnung mit 1-Brom-2-(trans-4-pentylcyclohexyl)-ethan/DMPU, Isolierung des Cyclohexylphenylethanderivates, erneute Metallierung mittels Butyllithium und anschliessende Reaktion mit festem Kohlendioxid erhält man 2,3-Difluor-4-[-2-(trans-4-pentylcyclohexyl)-ethyl]-benzoesäure. Diese wird wie üblich in das Amidinhydrochlorid überführt, das durch Umsetzung mit Hexylmalondialdehydtetramethylacetal 2-{2,3-Difluor-4-[2-(trans-4-pentylcyclohexyl)-ethyl]-phenyl}-5-hexylpyrimidin ergibt.

Beispiel 8:

Zu einer Lösung von 0,1 m 2,3-Difluornonyloxybenzol in 200 ml THF tropft man unter Rühren bei -60 bis -70 °C innerhalb einer halben Stunde eine Lösung von 0,1 m n-BuLi in Hexan. Man rührt noch 2 Stunden bei dieser Temperatur und tropft anschließend eine Lösung von 0,05 m $ZnBr_2$ (wasserfrei) in 100 ml THF bei gleicher Temperatur zu. Nach einer weiteren Stunde setzt man eine Lösung von 0,1 m 2-Brom-5-methylpyridin und 2 m% (0,002 m) Bis-triphenylphosphinnickel-II-chlorid, gelöst in 50 ml THF, langsam zu und rührt noch 16 Stunden nach, wobei die Temperatur langsam Raumtemperatur erreichen darf. Man setzt Wasser zu und arbeitet extraktiv auf. Das Rohprodukt wird durch umkristallisieren und chromatographieren gereinigt. Man erhält 2-(2,3-Difluor-4-nonyloxyphenyl)-5-methylpyridin.

Analog werden hergestellt:
2-(2,3-Difluor-4-nonyloxyphenyl)-5-octylpyridin
2-(2,3-Difluor-4-octyloxyphenyl)-5-octylpyridin, C 20 $S_c$ 26 N 37,9 I
2-(2,3-Difluor-4-octyloxyphenyl)-5-nonylpyridin
2-(2,3-Difluor-4-nonyloxyphenyl)-5-nonylpyridin
2-(2,3-Difluor-4-hexyloxyphenyl)-5-nonylpyridin

Beispiel 9:

Zu einer Lösung von 16,2 g 4-n-Octyl-2,3-difluorphenylboronsäure (hergestellt durch Lithiierung von 2,3-Difluoroctylbenzol, Umsetzung mit Trimethylborat und anschließende Hydrolyse des Boronsäureesters mit verdünnter Salzsäure) und 16,7 g 2-p-Bromphenyl-5-n-heptylpyrimidin in einem Gemisch aus 100 ml Toluol und 40 ml Ethanol gibt man 60 ml 2 m $Na_2CO_3$-Lösung und 0,5 g $Pd(PPh_3)_4$. Man erhitzt die Emulsion unter Rühren 18 Stunden zum Sieden, arbeitet die organische Phase wie üblich auf und erhält 2-(2,3-Difluor-4-n-octylbiphenyl-4′-yl)-5-n-heptylpyrimidin, K 64 $S_c$ 90 N 118 I.

Beispiel 10:

Aus 27,4 g 4-n-Pentyl-2,3-difluorphenylboronsäure und 7,5 g 2,5-Dichlorpyrazin erhält man analog Beispiel 9 2,5-Bis-(2,3-difluor-4-n-pentylphenyl)-pyrazin, K 117 N 120 I.

Beispiel 11:

Aus 2,7 g 2-Chlor-5-brompyrimidin (hergestellt durch Umsetzung von 2-Hydroxypyrimidin-hydrochlorid mit Brom in Wasser, Abdampfen des Wassers und Kochen des trockenen Rückstandes mit $POCl_3$) und 3,8 g 4-n-Octyl-2,3-difluorphenylboronsäure erhält man analog Beispiel 9 2-Chlor-5-(4-n-octyl-2,3-difluorphenyl)-pyrimidin, woraus man ebenfalls analog Beispiel 9 durch Umsetzung mit p-n-Pentylboronsäure 2-(p-n-Pentylphenyl)-5-(4-n-octyl-2,3-difluorphenyl)-pyrimidin erhält, K 82 $S_C$ 101 $S_A$ 105 N 121 I.

Beispiel 12:

0,1 mol 2-(2,3-Difluor-4-octyloxyphenyl)-5-hydroxypyrimidin (dargestellt durch Umsetzung von 2,3-Difluor-4-octyloxybenzamidin mit 2-Benzyloxytrimethiniumperchlorat [A. Holy u. Z. Arnold, Coll. Czech. Chem. Commun. 38, 1372 (1973)] und anschließende Hydrogenolyse des Benzylethers), 0.11 mol Kaliumcarbonat und 0,11 mol 1-Bromnonan werden in 100 ml Dimethylformamid unter Rühren 18 Stdn. auf 120° erhitzt. Man läßt abkühlen, filtriert die anorganischen Salze ab und destilliert vom Filtrat die Hauptmenge des Dimethylformamids ab. Die Lösung des Rückstandes in Dichlormethan wird mit Wasser gewaschen und getrocknet. Nach Abziehen des Lösungsmittels wird das 2-(2,3-Difluor-4-n-octyloxyphenyl)-5-n-nonyloxypyrimidin aus Ethanol umkristallisiert.

Analog erhält man, ausgehend von 2,3-Difluor-4-n-heptylbenzamidin, nach der gleichen Reaktionsfolge 2-(2,3-Difluor-4-n-heptylphenyl)-5-n-nonyloxypyrimidin.

Beispiel 13:

0,1 mol 2-(2,3-Difluor-4-nonylphenyl)-5-hydroxypyridin (hergestellt durch aufeinanderfolgende Umsetzung von 2,3-Difluor-4-nonylacetophenon mit 2-Benzyloxytrimethiniumperchlorat und Ammoniumacetat mit anschließender Hydrogenolyse des Benzylethers) werden mit 0,11 mol 1-Bromheptan und 0,11 mol Kaliumcarbonat in Dimethylformamid als Lösungsmittel verethert. Nach der Aufarbeitung wird das 2-(2,3-Difluor-4-nonylphenyl)-5-heptyloxypyridin aus Isopropanol umkristallisiert.

Analog erhält man durch Umsetzung von 2,3-Difluor-4-benzyloxyacetophenon mit 2-Octyloxytrimethiniumperchlorat, Ammoniumacetat und hydrierende Spaltung des Benzylethers 2-(2,3-Difluor-4-hydroxyphenyl)-5-octyloxypyridin, das mit 1-Bromdecan zum 2-(2,3-Difluor-4-n-decyloxyphenyl)-5-octyloxypyridin alkyliert wird.

Die folgenden Beispiele betreffen erfindungsgemäße flüssigkristalline Phasen:

Beispiel A

Eine flüssigkristalline Phase bestehend aus
8 % 2-p-Octyloxyphenyl-5-octylpyrimidin,
12 % 2-p-Nonyloxyphenyl-5-octylpyrimidin,
7 % 2-p-Hexyloxyphenyl-5-nonylpyrimidin,
23 % 2-p-Nonyloxyphenyl-5-nonylpyrimidin,
8 % 2-(2,3-Difluor-4-octyloxyphenyl)-5-nonyl-1,3,4-thiadiazol,
8 % 2-(2,3-Difluor-4-octyloxyphenyl)-5-heptyl-1,3,4-thiadiazol,
7 % 2-(p-Pentyloxyphenyl)-5-(p-heptylphenyl)-1,3,4-thiadiazol,
7 % 2-(p-Heptyloxyphenyl)-5-(p-heptylphenyl)-1,3,4-thiadiazol,
6 % 2-(p-Heptyloxyphenyl)-5-(2,3-difluor-4-heptylphenyl)-1,3,4-thiadiazol,
4 % 2-(4′-Heptyloxy-2,3-difluorbiphenyl-4-yl)-5-pentyl-1,3,4-thiadiazol und
10 % optisch aktivem 2-Cyan-2-methylhexansäure-(4′-octyloxybiphenyl-4-yl)-ester
zeigt $S_C^*$ 66 $S_A$ 70 Ch 80 I und eine Spontanpolarisation von 21 $nC/cm^2$ bei Raumtemperatur.

Beispiel B

Eine flüssigkristalline Phase bestehend aus
3 % 2-p-Heptyloxyphenyl-5-heptylpyrimidin,
4 % 2-p-Nonyloxyphenyl-5-heptylpyrimidin,
11 % 2-p-Octyloxyphenyl-5-nonylpyrimidin,
21 % 2-p-Nonyloxyphenyl-5-nonylpyrimidin,
7 % 2-(2,3-Difluor-4-octyloxyphenyl)-5-octylpyrimidin,
8 % 2-(2,3-Difluor-4-nonyloxyphenyl)-5-nonylpyrimidin,
4 % 2-(2,3-Difluor-4-octyloxyphenyl)-5-octylpyridin,
7 % 2-(p-Heptyloxyphenyl)-5-(p-pentylphenyl)-1,3,4-thiadiazol,
17 % r-1-Cyan-cis-4-(4′-octyloxybiphenyl-4-yl)-1-octylcyclohexan,
8 % 2-(4′-Pentyloxy-2,3-difluorbiphenyl-4-yl)-5-heptylpyrimidin und
10 % optisch aktivem 2-Chlor-3-methylbuttersäure-[p-(5-heptylpyrimidin-2-yl)-phenyl]-ester
zeigt $S_C^*$ 62 $S_A$ 66 Ch 78 I und eine Spontanpolarisation von 14 $nC/cm^2$ bei Raumtemperatur.

Beispiel C

Eine flüssigkristalline Phase bestehend aus:
7 % 2-p-Cyanphenyl-5-pentylpyrimidin,
8 % 2-p-Cyanphenyl-5-heptylpyrimidin,
8 % 2-p-Cyanphenyl-5-(p-butylphenyl)-pyrimidin,
5 % 4-Ethyl-4′-(trans-4-propylcyclohexyl)-biphenyl,
5 % 4-Ethyl-4′-(trans-4-pentylcyclohexyl)-biphenyl,
7 % 2-p-Methoxyphenyl-5-hexylpyrimidin,
6 % 2-p-Nonyloxyphenyl-5-heptylpyrimidin,
7 % 2-p-Methoxyphenyl-5-nonylpyrimidin,
6 % 2-(2,3-Difluor-4-hexyloxyphenyl)-5-nonylpyridin,
6 % 2-(2,3-Difluor-4-nonyloxyphenyl)-5-nonylpyrimidin,
7 % trans-4-Propylcyclohexancarbonsäure-(p-methoxyphenylester),
7 % trans-4-Propylcyclohexancarbonsäure-(p-ethoxyphenylester),
7 % trans-4-Butylcyclohexancarbonsäure-(p-methoxyphenylester),
7 % trans-4-Butylcyclohexancarbonsäure-(p-ethoxyphenylester) und
7 % trans-4-Pentylcyclohexancarbonsäure-(p-methoxyphenylester)
ist eine nematische Mischung mit hoher Doppelbrechung und günstigen High-Mux-Eigenschaften.

## Ansprüche

1. Heterocyclische Derivate des 1,2-Difluorbenzols der Formel I

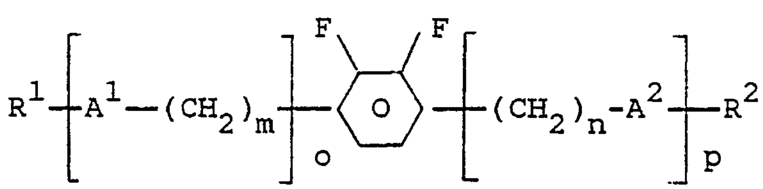

I

worin
$R^1$ und $R^2$ jeweils unabhängig voneinander eine Alkylgruppen mit 1-15 C-Atomen, worin auch eine oder mehrere $CH_2$-Gruppen durch einen bivalenten Rest ausgewählt aus der Gruppe bestehend aus -O-, -S-, -CO-, -O-CO-, -CO-O-, -CH=CH- und -C≡- ersetzt sein können, wobei zwei Heteroatome nicht direkt miteinander verknüpft sind,
$A^1$ und $A^2$ jeweils unabhängig voneinander eine unsubstituierte oder durch Fluor ein- oder mehrfach substituierte 1,4-Phenylengruppe, worin auch eine oder zwei CH-Gruppen durch N ersetzt sein können, eine 1,3,4-Thiadiazol-2,5-diyl-Gruppe oder eine trans-1,4-Cyclohexylen-Gruppe,

m und n jeweils 0 oder 2,
o 0 oder 1, und
p 0, 1 oder 2 bedeutet,
wobei (o + p) 1, 2 oder 3 ist und im Falle p = 2 die Gruppen $A^2$ und n gleich oder verschieden sein können, mit der Maßgabe, daß mindestens eine der Gruppen $A^1$ und $A^2$ eine 1,3,4-Thiadiazol-2,5-diyl-Gruppe oder eine unsubstituierte oder substituierte 1,4-Phenylengruppe ist, worin eine oder zwei CH-Gruppen durch N ersetzt sind.

2. Flüssigkristalline Phase mit mindestens zwei flüssigkristallinen Komponenten, dadurch gekennzeichnet, daß sie mindestens eine Verbindung der Formel I nach Anspruch 1 enthält.

3. Verwendung von Verbindungen der Formel I nach Anspruch 1 als Komponenten flüssigkristalliner Phasen.

4. Elektrooptisches Anzeigeelement, dadurch gekennzeichnet, daß es als Dielektrikum eine Phase nach Anspruch 2 enthält.

Europäisches Patentamt

# EUROPÄISCHER RECHERCHENBERICHT

Nummer der Anmeldung

EP 89 10 3496

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-3 703 651 (VEB WERK FÜR FERSEHELEKTRONIK GmbH)<br>* Insgesamt *<br>--- | 1-4 | C 07 D 285/12<br>C 07 D 239/26<br>C 07 D 213/30<br>C 07 D 213/65<br>C 09 K 19/34 |
| A | WO-A-8 607 055 (MERCK PATENT GmbH)<br>* Seiten 24-44,48-51; Ansprüche *<br>----- | 1-4 | |
| | | | **RECHERCHIERTE SACHGEBIETE (Int. Cl.4)** |
| | | | C 07 D 285/00<br>C 07 D 239/00<br>C 07 D 213/00<br>C 09 K 19/00 |

Der vorliegende Recherchenbericht wurde für alle Patentansprüche erstellt

| Recherchenort | Abschlußdatum der Recherche | Prüfer |
|---|---|---|
| DEN HAAG | 08-05-1989 | BESLIER L.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)